(19) Europäisches Patentamt
European Patent Office
Office européen des brevets

(11) Numéro de publication: **0 461 958 B1**

(12) **FASCICULE DE BREVET EUROPEEN**

(45) Date de publication de fascicule du brevet: **14.09.94**

(51) Int. Cl.5: **C07D 319/06**, A61K 31/335

(21) Numéro de dépôt: **91401461.8**

(22) Date de dépôt: **05.06.91**

# (54) Dérivés de 2-(aminoalkyl)-5-(arylalkyl)-1,3-dioxanes, leur préparation et leur application en thérapeutique.

(30) Priorité: **15.06.90 FR 9007483**
**15.04.91 FR 9104578**

(43) Date de publication de la demande:
**18.12.91 Bulletin 91/51**

(45) Mention de la délivrance du brevet:
**14.09.94 Bulletin 94/37**

(84) Etats contractants désignés:
**AT BE CH DE DK ES FR GB GR IT LI LU NL SE**

(56) Documents cités:
**FR-A- 1 554 878**
**US-A- 4 097 491**
**US-A- 4 116 670**

**CHEMICAL ABSTRACTS, vol. 104, no. 21, 26 mai 1986 Columbus, Ohio, USA Swinyard,Ewart A. et al.: "Comparative anticonvulsant activity and neurotoxicity of felbamate and four prototype antiepileptic drugs & Epilepsia N.Y 1986, 27 1 ,27-34 page 50; colonne 2; ref. no. 180056V EP 91401461030 &en liaison avec CA vol.71 Registry Number Index, page 1139N.**

(73) Titulaire: **SYNTHELABO**
**22, Avenue Galilée**
**F-92350 Le Plessis Robinson (FR)**

(72) Inventeur: **Raizon, Bernard**
**49, rue Gabriel Péri**
**F-91270 Vigneux (FR)**
Inventeur: **Evanno, Yannick**
**20, rue Oudry**
**F-75013 Paris (FR)**
Inventeur: **Legalloudec, Odette**
**1, rue au Comte**
**F-91150 Morigny (FR)**

(74) Mandataire: **Ludwig, Jacques et al**
**SYNTHELABO,**
**Service Brevets,**
**B.P. 72**
**F-92352 Le Plessis Robinson Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 461 958 B1

## Description

La présente invention a pour objet des dérivés de 2-(Aminoalkyl)-5-(arylalkyl)-1,3-dioxanes, leur préparation et leur application en thérapeutique.

Les composés de l'invention répondent à la formule générale (I), donnée dans le schéma de réaction ci-après, formule dans laquelle
m représente 0 ou 1,
n représente 1, 2, 3 ou 4,
$R_1$ représente soit un atome d'hydrogène soit un groupe méthyle,
$R_2$ représente soit un atome d'hydrogène, soit un groupe méthyle, soit un groupe alcanoyle de formule générale COR′ dans laquelle R′ représente un atome d'hydrogène ou un groupe alkyle en $C_1$-$C_4$ linéaire ou ramifié, soit un groupe alcoxycarbonyle de formule générale COOR″ dans laquelle R″ représente un groupe alkyle en $C_1$-$C_4$ linéaire ou ramifié, et Ar représente soit un groupe phényle portant éventuellement un ou deux substituants choisis parmi les atomes d'halogène et les groupes alkyle en $C_1$-$C_4$, méthoxy et trifluorométhyle, soit un groupe naphtalén-1-yle ou naphtalén-2-yle portant éventuellement un substituant choisi parmi les atomes d'halogène et les groupes méthyle, méthoxy et cyclopropylméthoxy.

Les composés de formule générale (I) peuvent exister sous forme de stéréoisomères cis ou trans, et aussi sous forme de bases libres ou de sels d'addition à des acides ; ces diverses formes font partie de l'invention.

Conformément à l'invention, on peut préparer les composés de formule générale (I) par un procédé illustré par le schéma ci-après.
Il consiste à faire réagir un diol de formule générale (II) (dans laquelle m et Ar sont tels que définis ci-dessus) avec un acétal de formule générale (III) (dans laquelle n, $R_1$ et $R_2$ sont tels que définis ci-dessus et R représente un groupe alkyle en $C_1$-$C_4$), dans un solvant tel que l'éther diéthylique ou le benzène, à une température de 20 à 80 °C, selon le solvant utilisé, et en présence d'acide paratoluènesulfonique et/ou d'éther chlorhydrique comme catalyseur.

Schéma

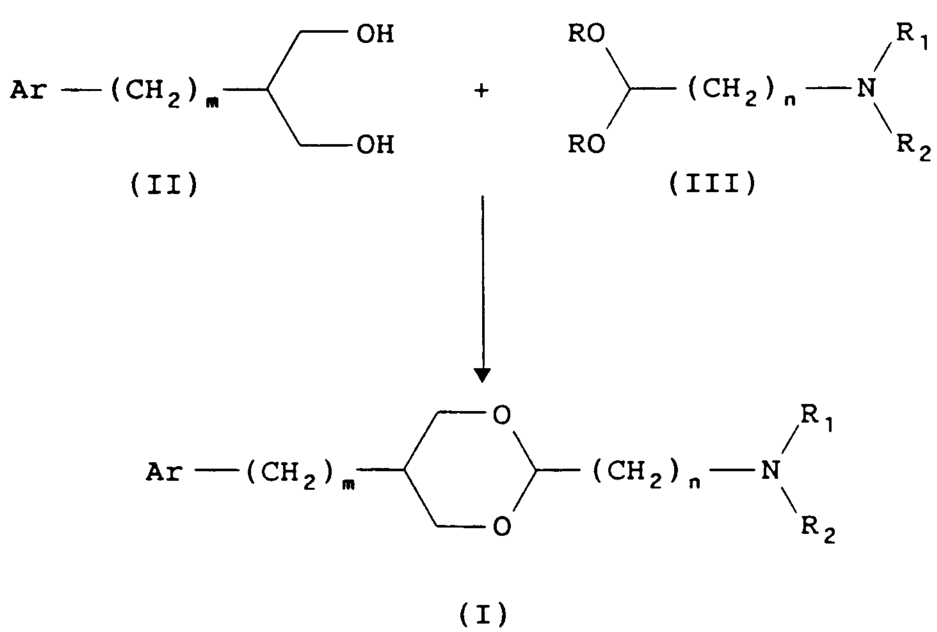

Les diols de formule générale (II) sont largement décrits dans la littérature et peuvent être préparés selon toutes méthodes connues.
Ainsi, lorsque m = 0, on peut par exemple estérifier un acide de formule générale Ar-$CH_2$-COOH par

l'éthanol en présence de chlorure de thionyle, faire réagir l'ester de formule générale $ArCH_2COOC_2H_5$ avec le carbonate d'éthyle en présence d'éthylate de sodium, et finalement réduire le diester de formule générale $ArCH(COOC_2H_5)_2$ au moyen de borohydrure de sodium. Lorsque Ar représente un groupe naphtalényle, l'ester de formule générale $ArCH_2COOC_2H_5$ peut également être obtenu à partir de la 1,2,3,4-tétrahydronaphtalénone correspondante selon un procédé dont le détail est donné dans les exemples. Lorsque m = 1, on peut par exemple faire réagir un dérivé halogéné de formule générale $ArCH_2Cl$ avec le malonate de diéthyle en présence d'éthylate de sodium, et finalement réduire le diester de formule générale $ArCH_2CH(COOC_2H_5)_2$ au moyen d'hydrure de lithium et d'aluminium.

Les acétals de formule générale (III) sont également bien décrits dans la littérature, et peuvent aussi être préparés selon les méthodes connues ; ainsi, par exemple, ceux dans la formule générale desquels $R_1$ et/ou $R_2$ sont différents d'un atome d'hydrogène, peuvent être préparés à partir d'une amine de formule générale (III) dans laquelle $R_1$ et $R_2$ représentent chacun un atome d'hydrogène selon toutes méthodes connues d'alkylation ou de préparation d'amides ou de carbamates.

Ces mêmes méthodes peuvent d'ailleurs servir à transformer un composé de formule générale (I) dans laquelle $R_1$ et $R_2$ représentent chacun un atome d'hydrogène en un autre composé dans la formule duquel $R_1$ et/ou $R_2$ sont différents d'un atome d'hydrogène.

Les exemples suivants illustrent en détail la préparation de quelques composés selon l'invention. Les microanalyses élémentaires et les spectres IR et RMN confirment les structures des produits obtenus.
Les numéros donnés entre parenthèses dans les titres des exemples correspondent à ceux du tableau qui suit.

Exemple 1 (Composé N° 58)

*N*-[5-(4-chlorophényl)méthyl-1,3-dioxan-2-yl]méthylacétamide.

1.1 2-(4-Chlorophényl)méthylpropanedioate de diéthyle.
On ajoute une solution d'éthylate de sodium (2,86 g (124 mmoles) de sodium dans 50 ml d'éthanol) à une solution de 20 g (124 mmoles) de 1-chloro-4-(chlorométhyl)benzène et de 19,9 g (124 mmoles) de malonate de diéthyle dans 50 ml d'éthanol. On chauffe au reflux pendant 4h, on évapore le solvant sous pression réduite, et on reprend le résidu avec 250 ml de chloroforme. On lave la phase organique avec une solution saturée d'hydrogénocarbonate de sodium, on la sèche sur sulfate de magnésium, on l'évapore, et on distille le résidu sous pression réduite (80-85 °C, 4 Pa, soit 0,03 mmHg). On obtient 16,8 g (59 mmoles) de produit.
1.2. 2-[(4-Chlorophényl)méthyl]propane-1,3-diol.
On ajoute, goutte à goutte, une solution de 16,8 g (59 mmoles) de 2-(4-chlorophényl)-méthylpropanedioate de diéthyle dans 50 ml de tétrahydrofuranne à une suspension de 5 g (131 mmoles) d'hydrure de lithium et d'aluminium dans 500 ml de tétrahydrofuranne. Après chauffage au reflux pendant 8h on hydrolyse le mélange avec 5 g d'eau, 15 g d'hydroxyde de sodium à 10% puis 5 g d'eau, puis on l'évapore sous pression réduite. On reprend le résidu avec 200 ml de dichlorométhane, et on lave la solution trois fois avec de l'eau, on la sèche sur sulfate de magnésium, on la filtre, on évapore le filtrat et on ajoute un peu d'éther diéthylique et de pentane au résidu. On filtre le précipité obtenu et on le sèche sous vide. On obtient 9,1 g (45 mmoles) de produit. Point de fusion : 58-60 °C.
1.3. *N*-(2,2-diméthoxyéth-1-yl)acétamide.
A une suspension, agitée et chauffée au reflux, de 40 g (290 mmoles) de carbonate de potassium et de 30 g (285 mmoles) d'aminoacétaldéhyde diméthylacétal dans 200 ml de benzène on ajoute, goutte à goutte, une solution de 32 g (313 mmoles) d'anhydride acétique dans 200 ml de benzène. On maintient le reflux pendant 30mn puis on refroidit le mélange, on le filtre, on lave le précipité avec du benzène, on évapore le filtrat, et on traite l'huile résiduelle avec du charbon végétal dans 300 ml d'éther diéthylique. Après filtration et évaporation on obtient 36,8 g (250 mmoles) de produit.
$n_D^{25} = 1,4450$.
1.4. *N*-[5-(4-chlorophényl)méthyl-1,3-dioxan-2-yl]méthylacétamide.
A 150 ml d'éther diéthylique on ajoute 4 g (20 mmoles) de 2-[(4-chlorophényl)méthyl]propane-1,3-diol, 2,94 g (20 mmoles) de *N*-(2,2-diméthoxyéth-1-yl)acétamide, 0,3 g d'acide paratoluènesulfonique et 4 ml d'éther diéthylique saturé d'acide chlorhydrique. On évapore le mélange sous vide puis, à deux reprises, on ajoute au résidu 150 ml de benzène, on chauffe à 70-80 °C sous un léger vide et on évapore le solvant. On refroidit le résidu, on ajoute 150 ml de dichlorométhane, on lave la solution avec une solution saturée d'hydrogénocarbonate de sodium puis avec de l'eau et on la sèche sur sulfate de magnésium. On purifie le résidu deux fois par chromatographie sur colonne de gel de silice en éluant avec du

chloroforme, et finalement on le recristallise dans un mélange 70/4 de cyclohexane/acétate d'éthyle. On obtient 1,7 g (6 mmoles) de produit.
Point de fusion : 95 ° C.

Exemple 2 (Composé N ° 120)

5-(Naphtalén-1-yl)-1,3-dioxan-2-propanamine, chlorhydrate.

A 4 g (25 mmoles) de 4,4-diéthoxybutan-1-amine dans 500 ml de benzène on ajoute assez d'éther diéthylique chlorhydrique pour précipiter tout le chlorhydrate. On ajoute alors 4 g (20 mmoles) de 2-(naphtalén-1-yl)propane-1,3-diol dans 200 ml de benzène et 100 mg d'acide paratoluènesulfonique, et on évapore le mélange à sec dans un bain à 100 ° C. On ajoute encore 700 ml de benzène, on l'évapore dans les mêmes conditions, puis on place le résidu sous vide à 100 ° C pendant 4h. On le recristallise dans l'éther diéthylique, on le sèche à 60 ° C, on le recristallise deux fois dans l'éthanol en le traitant au charbon végétal lors de la première recristallisation, on le lave à l'éther diéthylique et on le sèche à 80 ° C. On obtient 3,2 g (10,4 mmoles) de produit.
Point de fusion : 191-192 ° C.

Exemple 3 (Composé N ° 121)

*N*-Méthyl-5-(naphtalén-1-yl)-1,3-dioxan-2-propanamine, chlorhydrate.

3.1. *N*-[3-[5-(Naphtalén-1-yl)-1,3-dioxan-2-yl]propyl]-2,2,2-trifluoroacétamide.
On chauffe pendant 3h, dans un appareil de Dean-Stark, 20 g (65 mmoles) de chlorhydrate de 5-(naphtalén-1-yl)-1,3-dioxan-2-propanamine et 19,5 g (141 mmoles) de carbonate de potassium dans 350 ml de benzène. Puis on introduit 14 g (67 mmoles) d'anhydride trifluoroacétique et on chauffe encore pendant 3h. On refroidit le mélange, on le filtre et on évapore le filtrat à sec. Le résidu cristallise dans le pentane. On obtient 15 g (41 mmoles) de produit brut. Point de fusion : 83-84 ° C.
3.2. *N*-Méthyl-5-(naphtalén-1-yl)-1,3-dioxan-2-propanamine, chlorhydrate.
On dissout 12 g (33 mmoles) du produit brut obtenu dans l'étape précédente dans 200 ml d'acétone et on ajoute, goutte à goutte et tout en agitant, 20,7 g (146 mmoles) d'iodométhane, et on chauffe jusqu'au reflux. On ajoute alors 8,2 g (146 mmoles) d'hydroxyde de potassium pulvérisé, et on maintient le reflux pendant 15mn. On évapore le solvant, on ajoute 90 ml d'eau au résidu, on le chauffe au reflux pendant 10mn, on refroidit le mélange, et on l'extrait trois fois à l'éther. On sèche la phase éthérée sur sulfate de magnésium, on la filtre, et on ajoute au filtrat suffisamment d'éther diéthylique saturé d'acide chlorhydrique pour précipiter le chlorhydrate. On purifie ce dernier en le recristallisant deux fois dans un mélange 50/50 de méthanol/éther diéthylique. Après lavage à l'éther diéthylique et séchage à 70 ° C on isole finalement 6,9 g (21,4 mmoles) de produit. Point de fusion : 142-144 ° C.

Exemple 4 (Composé N ° 123)

*N*-[3-[5-(Naphtalén-1-yl)-1,3-dioxan-2-yl]propyl]acétamide.

4.1. Naphtaléne-1-acétate d'éthyle.
On chauffe à 60 ° C, tout en agitant, 100 g (537 mmoles) d'acide naphtalène-1-acétique dans 200 ml d'éthanol absolu, et on ajoute lentement, de façon à obtenir le reflux, 80 ml de chlorure de thionyle. On maintient le reflux pendant 6h, puis on évapore le solvant sous pression réduite. On reprend le résidu avec 200 ml de dichlorométhane, on lave la solution avec une solution saturée d'hydrogénocarbonate de sodium puis avec de l'eau, on la sèche sur sulfate de magnésium, on la filtre et on évapore le solvant sous pression réduite. On obtient 104 g (485 mmoles) de produit sous forme d'huile.
$n_D^{25} = 1,5800$.
4.2. 2-(Naphtalén-1-yl)propanedioate de diéthyle.
On agite à 105 ° C un mélange de 103,9 g (485 mmoles) de naphtaléne-1-acétate d'éthyle et de 430 ml de carbonate d'éthyle, puis on ajoute une solution d'éthylate de sodium (11 g (478 mmoles) de sodium dans 255 ml d'éthanol absolu) de façon à distiller l'éthanol au cours de l'introduction. On chauffe de nouveau pendant 15mn pour distiller le maximum d'éthanol, on refroidit rapidement le ballon et on verse le mélange sur 300 g de glace pilée et 20 ml d'acide chlorhydrique concentré. On l'extrait à l'éther diéthylique, on sèche la phase organique sur sulfate de magnésium, on la filtre et on évapore le solvant

sous pression réduite. On obtient une huile qui cristallise à froid dans le pentane. On sépare les cristaux par filtration, on les traite au charbon végétal dans l'éther diéthylique, on filtre, on évapore le filtrat et on recristallise le résidu dans un mélange 25/75 d'éther diéthylique/pentane. On obtient 97 g (339 mmoles) de cristaux. Point de fusion : 57-59 °C.

4.3. 2-(Naphtalén-1-yl)propane-1,3-diol.

On agite à température ambiante 50 g (175 mmoles) de 2-(naphtalén-1-yl)propanedioate de diéthyle dans 310 ml de dioxane et 300 ml d'eau; Puis on ajoute, par portions, un excès (2,2 moles, soit 84 g) de borohydrure de sodium; On agite pendant 8h, et on laisse les produits en contact pendant 2 jours. Puis on hydrolyse le mélange, sous agitation, en introduisant de la glace pilée et de l'acide chlorhydrique 3N jusqu'à obtenir un pH acide. On extrait le mélange à l'éther diéthylique, on évapore la phase organique, et on purifie l'huile résiduelle par chromatographie sur colonne de gel de silice en éluant avec de l'acétate d'éthyle. Après évaporation et séchage à 40 °C en présence d'anhydride phosphorique on isole 18,5 g (91 mmoles) de produit. Point de fusion : 58-60 °C.

4.4. *N*-(4,4-Diéthoxybut-1-yl)acétamide.

A une suspension agitée de 32 g (198 mmoles) de 4,4-diéthoxybutan-1-amine et de 24 g (174 mmoles) de carbonate de potassium dans 600 ml de benzène on ajoute, goutte à goutte, au reflux, 20 g (196 mmoles) d'anhydride acétique dans 100 ml de benzène. On chauffe le mélange au reflux pendant 15mn, on le refroidit, on filtre le précipité et on évapore le filtrat à sec. On traite l'huile résiduelle avec du charbon végétal dans 300 ml d'éther diéthylique et, après filtration et évaporation du filtrat sous vide on obtient 37,3 g (183 mmoles) de produit huileux. $n_D^{20}$ = 1,4472.

4.5. *N*-[3-[5-(Naphtalén-1-yl)-1,3-dioxan-2-yl]propyl]acétamide.

A un mélange de 11,6 g (57 mmoles) de 2-(naphtalén-1-yl)propane-1,3-diol et de 11,65 g de *N*-(4,4-diéthoxybut-1-yl)acétamide dans 300 ml d'éther diéthylique on ajoute 10 ml d'éther chlorhydrique et 100 mg d'acide paratoluènesulfonique, et on évapore le mélange à sec dans un bain à 50 °C. Puis, à deux reprises, on ajoute 300 ml de benzène et on évapore le mélange à sec dans un bain à 70 °C. On place le résidu sous vide de la trompe à eau pendant 45mn, on reprend le résidu avec 300 ml de dichlorométhane, et on lave la solution avec une solution saturée d'hydrogénocarbonate de sodium puis avec de l'eau. On la sèche sur sulfate de magnésium, on la filtre et on l'évapore à sec. On recristallise le résidu dans un mélange 80/20 d'acétate d'éthyle/dichlorométhane, en le traitant avec du charbon végétal. Après une deuxième recristallisation, lavage à l'éther diéthylique et séchage à 60 °C on isole finalement 9 g (29 mmoles) de produit.

Point de fusion : 158-160 °C.

Exemple 5 (Composé N° 125)

[3-[5-(Naphtalén-1-yl)-1,3-dioxan-2-yl]propyl]carbamate d'éthyle.

On agite à température ambiante, pendant 10mn, 4 g (13 mmoles) de chlorhydrate de 5-(naphtalén-1-yl)-1,3-dioxan-2-propanamineavec 4 ml de triéthylamine dans 200 ml de dichlorométhane, puis on ajoute, goutte à goutte, 1,4 g (13 mmoles) de chloroformiate d'éthyle dans 20 ml de dichlorométhane, et on agite le mélange pendant 3h à température ambiante. On ajoute de l'acide chlorhydrique dilué, on sépare la phase organique, on la lave avec de l'eau et avec une solution saturée d'hydrogénocarbonate de sodium, on la sèche sur sulfate de magnésium, on la filtre et on l'évapore à sec. On obtient une huile qui cristallise dans le pentane; On filtre le solide et on le purifie par chromatographie sur colonne de gel de silice en éluant avec de l'acétate d'éthyle. On isole finalement 2,4 g (7 mmoles) de produit.
Point de fusion : 93-94 °C.

Exemple 6 (Composé N° 128)

*N*-Méthyl-*N*-[3-[5-(naphtalén-1-yl)-1,3-dioxan-2-yl]propyl]acétamide.

On chauffe à 60 °C, tout en agitant, un mélange de 3 g (9,3 mmoles) de chlorhydrate de *N*-méthyl-5-(naphtalén-1-yl)-1,3-dioxan-2-propanamine avec 2,45 g (18 mmoles) de carbonate de potassium dans 100 ml de benzène. On ajoute 1 g (10 mmoles) d'anhydride acétique et on chauffe le mélange au reflux pendant 30mn; On le refroidit, on le filtre, on lave le filtrat à l'eau, on le sèche sur sulfate de magnésium, on le filtre et on évapore le filtrat à sec. On reprend le résidu avec de l'éther diéthylique, on le traite au charbon végétal, on filtre, on évapore le filtrat. Après séchage à 50 °C on obtient 1,3 g (4 mmoles) de produit huileux.

$n_D^{21}$ = 1,5752

Exemple 7 (Composé N° 136)

*N*-[5-(6-Méthoxynaphtalén-1-yl)-1,3-dioxan-2-yl]méthylacétamide.

7.1. 6-Méthoxynaphtaléne-1-acétate d'éthyle.

Dans un réacteur de 2 l contenant 500 ml de tétrahydrofurane on place, sous argon, 50 g (284 mmoles) de 6-méthoxy-1,2,3,4-tétrahydronaphtalén-1-one, 62 g (371 mmoles) de bromoacétate d'éthyle, 5 g (39 mmoles) d'iode et 33 g (500 mmoles) de poudre de zinc, et on chauffe le mélange sur bain d'huile de façon à initier la réaction. Lorsque la température atteint 50°C environ, la réaction démarre et on augmente l'agitation pour détruire la mousse qui se forme. Le mélange prend une coloration verdâtre, et on le laisse au reflux pendant 1h. On le refroidit, on ajoute un solution d'hydrogénosulfate de potassium pour ajuster le pH entre 1 et 2, on chauffe le mélange au reflux encore pendant 1h, puis on le filtre et on extrait le filtrat avec de l'éther diéthylique. Après séchage et évaporation de la phase organique on obtient 72 g d'ester brut qui est un mélange de 6-méthoxy-3,4-dihydronaphtalène-1-acétate d'éthyle et de (6-méthoxy-1,2,3,4-tétrahydronaphtalène-1,1-diyl)acétate d'éthyle.

On en dissout 70 g (284 mmoles) dans 500 ml de benzène, on ajoute 74 g (292 mmoles) de 2,3-dichloro-5,6-dicyanocyclohexa-2,5-diène-1,4-dione et on chauffe le mélange pendant 6h au reflux. On filtre le mélange, on évapore le filtrat et on purifie le résidu par chromatographie sur colonne de gel de silice en éluant avec un mélange 95/5 d'hexane/acétate d'éthyle, et on isole 39,4 g d'ester (161 mmoles).

7.2. 2-(6-Méthoxynaphtalén-1-yl)propanedioate de diéthyle.

On chauffe à 110°C, au bain d'huile, 14 g (57 mmoles) de 6-méthoxynaphtaléne-1-acétate d'éthyle en solution dans 46 ml de carbonate de diéthyle, on ajoute une solution fraîchement préparée d'éthylate de sodium (1,45 g de sodium dans 30 ml d'éthanol, soit 63 mmoles) tout en distillant l'alcool, en 45 minutes.

On maintient le chauffage pendant 1h, puis on hydrolyse le mélange avec 100 ml d'eau et de l'acide chlorhydrique aqueux jusqu'à pH = 1, on l'extrait deux fois avec 150 ml de dichlorométhane. Après séchage et évaporation de la phase organique on obtient 20 g de produit brut qu'on purifie par chromatographie sur colonne de gel de silice en éluant avec un mélange d'hexane/éther diéthylique d'abord 95/5 puis 80/20. On isole 14,5 g de diester (46 mmoles).

7.3. 2-(6-Méthoxynaphtalén-1-yl)propane-1,3-diol.

On dissout 31 g (98 mmoles) de 2-(6-méthoxynaphtalén-1-y1)propanedioate de diéthyle dans 150 ml de tétrahydrofurane, et on ajoute la solution à 7,5 g (197 mmoles) d'hydrure de lithium et d'aluminium en suspension dans 150 ml de tétrahydrofurane.

On chauffe le mélange au reflux pendant 6h, on le refroidit et on l'hydrolyse en ajoutant successivement 7,5 g d'eau, 21 g de soude à 10% et 7,5 g d'eau. On agite le mélange pendant 1h à environ 50°C, on le filtre, en lavant le solide avec du tétrahydrofurane chaud, on concentre le filtrat sous pression réduite, on reprend le résidu avec du chloroforme, on lave la solution deux fois à l'eau, on la sèche et on évapore le solvant. On purifie le résidu par chromatographie sur colonne de gel de silice en éluant d'abord avec un mélange 1/1 d'hexane/acétate d'éthyle, puis avec de l'acétate d'éthyle pur. On isole 9,5 g de diol (41 mmoles).

7.4. *N*-[5-(6-Méthoxynaphtalén-1-yl)-1,3-dioxan-2-yl]méthylacétamide.

Dans un ballon contenant 200 ml de benzène on introduit 2,3 g (10 mmoles) de 2-(6-méthoxynaphtalén-1-yl)propane-1,3-diol, 0,45 g d'acide paratoluènesulfonique et 1,6 g (11 mmoles) de *N*-(2,2-diméthoxyéth-1-yl)acétamide.

On chauffe le mélange à 60°C pendant 1h, on évapore le solvant sous pression réduite, on reprend le résidu avec 200 ml de benzène, on chauffe de nouveau pendant 1h à 60°C et on évapore le solvant sous pression réduite. On purifie le résidu par chromatographie sur colonne de gel de silice en éluant avec un mélange 1/1 de chloroforme/acétate d'éthyle. Après recristallisation dans l'acétate d'éthyle on isole finalement 1,2 g de composé (3,8 mmoles).

Point de fusion : 158°C.

Exemple 8 (Composé N° 137)

[[5-(6-Méthoxynaphtalén-1-yl)-1,3-dioxan-2-yl]méthyl]carbamate de méthyle.

On chauffe au reflux pendant 3h un mélange de 200 ml de benzène, 3 g (13 mmoles) de 2-(6-méthoxynaphtalén-1-yl)propane-1,3-diol, 1,5 g (14 mmoles) de 2,2-diméthoxyéthanamine et 50 ml d'éther diéthylique saturé d'acide chlorhydrique gazeux.
On évapore les solvants sous pression réduite, on reprend le résidu avec du chloroforme, on ajoute une solution de carbonate de potassium, on sépare la phase organique, on la sèche et on l'évapore. On reprend le résidu avec 100 ml de chloroforme, on ajoute 2,6 g (26 mmoles) de triéthylamine puis, au goutte à goutte, 1,35 g (14 mmoles) de chloroformiate de méthyle, et on laisse le mélange sous agitation à température ambiante pendant plusieurs heures.
On lave la phase organique en milieu acide, puis en milieu basique, on la sèche et on l'évapore sous pression réduite. On purifie l'huile résiduelle par chromatographie sur colonne de gel de silice en éluant avec un mélange 99/1 de chloroforme/éthanol. Après recristallisation dans l'acétate d'éthyle on isole finalement 1,9 g de composé pur (5,7 mmoles).
Point de fusion : 142-144° C.

Exemple 9 (Composé N° 138)

*N*-[3-[5-(6-Méthoxynaphtalén-1-yl)-1,3-dioxan-2-yl]propyl]acétamide (de conformation trans diéquatorial).

9.1. *N*-(4,4-Diéthoxybut-1-yl)acétamide.
A une suspension agitée de 32 g (198 mmoles) de 4,4-diéthoxybutan-1-amine et de 24 g (174 mmoles) de carbonate de potassium dans 600 ml de benzène on ajoute, goutte à goutte, au reflux, 20 g (196 mmoles) d'anhydride acétique dans 100 ml de benzène. On chauffe le mélange au reflux pendant 15mn, on le refroidit, on filtre le précipité et on évapore le filtrat à sec. On traite l'huile résiduelle avec du charbon végétal dans 300 ml d'éther diéthylique et, après filtration et évaporation du filtrat sous vide on obtient 37,3 g de produit huileux. $n_D^{20} = 1{,}4472$.

9.2. *N*-[3-[5-(6-Méthoxynaphtalén-1-yl)-1,3-dioxan-2-yl]propyl]acétamide.
Dans un ballon contenant 300 ml de benzène on introduit 9,5 g (41 mmoles) de 2-(6-méthoxynaphtalén-1-yl)propane-1,3-diol, 1 g d'acide paratoluènesulfonique et 9,2 g (45 mmoles) de *N*-(4,4-diéthoxybut-1-yl)acétamide.
On agite le mélange pendant 2h à 50° C, on l'évapore à sec, on ajoute 300 ml de benzène, et on renouvelle l'opération. On ajoute encore une fois 300 ml de benzène, ainsi que 1,4 g (7 mmoles) de *N*-(4,4-diéthoxybut-1-yl)acétamide et on chauffe le mélange de nouveau à 50° C pendant 1h.
On évapore le solvant sous pression réduite et on purifie le résidu par chromatographie sur colonne de gel de silice en éluant avec de l'acétate d'éthyle. Après traitement au charbon végétal on obtient 11,6 g de produit qu'on recristallise deux fois dans l'acétate d'éthyle. On isole finalement 7,6 g de composé pur (22 mmoles).
Point de fusion : 146° C.

Le tableau ci-après illustre les structures chimiques et les propriétés physiques de quelques composés selon l'invention. Dans la colonne "Ar", $C_6H_x$ désigne un groupe phényle portant 5-x substituants, $C_{10}H_{7-x}$-(1)- et $C_{10}H_{7-x}$-(2)- désignent des groupes naphtalén-1-yle et naphtalén-2-yle, respectivement, portant 7-x substituants, et $cC_3H_5$ désigne un groupe cyclopropyle.
Dans la colonne "$R_2$", $C_2H_5$, $iC_3H_7$, $iC_4H_9$ et $tC_4H_9$ désignent les groupe éthyle, isopropyle, isobutyle et tertiobutyle, respectivement.
Dans la colonne "T/C" sont indiqués les rapports trans/cis des composés.

Tableau

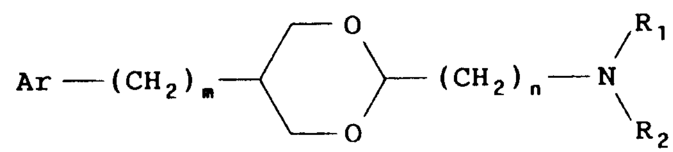

| N° | Ar | m | n | $R_1$ | $R_2$ | Sel | T/C | F (°C) |
|---|---|---|---|---|---|---|---|---|
| 1 | $C_6H_5$- | 0 | 2 | $CH_3$ | $CH_3$ | HCl | 100/0 | 96-98 |
| 2 | $C_6H_5$- | 0 | 3 | H | H | HCl | 100/0 | 175-176 |
| 3 | $C_6H_5$- | 0 | 3 | H | $CH_3$ | HCl | 100/0 | 188-190 |
| 4 | $C_6H_5$- | 0 | 3 | H | $COCH_3$ | base | 100/0 | 103-104 |
| 5 | $C_6H_5$- | 0 | 3 | H | $COC_2H_5$ | base | 100/0 | 92-93 |
| 6 | $C_6H_5$- | 0 | 3 | H | $COtC_4H_9$ | base | 100/0 | 114-115 |
| 7 | $C_6H_5$- | 1 | 1 | H | $COCH_3$ | base | 55/45 | $n_D^{20}$ =1,5280 |
| 8 | $C_6H_5$- | 1 | 2 | H | $COCH_3$ | base | 85/15 | 96 |
| 9 | $C_6H_5$- | 1 | 3 | H | $COCH_3$ | base | 60/40 | 72 |
| 10 | 2-F-$C_6H_4$- | 1 | 1 | H | $COCH_3$ | base | 60/40 | 84-86 |

Tableau (suite)

| N° | Ar | m | n | $R_1$ | $R_2$ | Sel | T/C | F (°C) |
|---|---|---|---|---|---|---|---|---|
| 11 | $2-F-C_6H_4-$ | 1 | 2 | H | $COCH_3$ | base | 90/10 | 94-96 |
| 12 | $2-F-C_6H_4-$ | 1 | 3 | H | $COCH_3$ | base | 90/10 | 83-85 |
| 13 | $2-F-C_6H_4-$ | 1 | 3 | H | $COOC_2H_5$ | base | 100/0 | 96-98 |
| 14 | $3-F-C_6H_4-$ | 1 | 1 | H | $COCH_3$ | base | 90/10 | 94-96 |
| 15 | $3-F-C_6H_4-$ | 1 | 2 | H | $COCH_3$ | base | 85/15 | 90-91 |
| 16 | $3-F-C_6H_4-$ | 1 | 3 | H | $COCH_3$ | base | 80/20 | 76-78 |
| 17 | $4-F-C_6H_4-$ | 0 | 1 | H | $COCH_3$ | base | 100/0 | 93-95 |
| 18 | $4-F-C_6H_4-$ | 0 | 1 | H | $COOCH_3$ | base | 100/0 | 87-89 |
| 19 | $4-F-C_6H_4-$ | 0 | 1 | $CH_3$ | $COCH_3$ | base | 75/25 | 46-48 |
| 20 | $4-F-C_6H_4-$ | 0 | 1 | $CH_3$ | $COOCH_3$ | base | 100/0 | 43-45 |
| 21 | $4-F-C_6H_4-$ | 0 | 2 | H | $COCH_3$ | base | 100/0 | 142-143 |
| 22 | $4-F-C_6H_4-$ | 0 | 2 | H | $COOCH_3$ | base | 100/0 | 92-94 |
| 23 | $4-F-C_6H_4-$ | 0 | 3 | H | $COCH_3$ | base | 100/0 | 100-102 |
| 24 | $4-F-C_6H_4-$ | 0 | 3 | H | $COOCH_3$ | base | 100/0 | 70-72 |
| 25 | $4-F-C_6H_4-$ | 1 | 1 | H | $COCH_3$ | base | 90/10 | 119-120 |
| 26 | $4-F-C_6H_4-$ | 1 | 2 | H | $COCH_3$ | base | 95/5 | 136-137 |

Tableau (suite)

| N° | Ar | m | n | $R_1$ | $R_2$ | Sel | T/C | F (°C) |
|---|---|---|---|---|---|---|---|---|
| 27 | $4-F-C_6H_4-$ | 1 | 3 | H | $COCH_3$ | base | 90/10 | 118-120 |
| 28 | $2-Cl-C_6H_4-$ | 0 | 1 | H | $COCH_3$ | base | 100/0 | 104-106 |
| 29 | $2-Cl-C_6H_4-$ | 0 | 2 | H | $COCH_3$ | base | 100/0 | 100-102 |
| 30 | $2-Cl-C_6H_4-$ | 0 | 3 | H | $COCH_3$ | base | 100/0 | 118-120 |
| 31 | $2-Cl-C_6H_4-$ | 1 | 1 | H | $COCH_3$ | base | 90/10 | 110-111 |
| 32 | $2-Cl-C_6H_4-$ | 1 | 2 | H | $COCH_3$ | base | 70/30 | 89-91 |
| 33 | $2-Cl-C_6H_4-$ | 1 | 3 | H | $COCH_3$ | base | 60/40 | 62-64 |
| 34 | $3-Cl-C_6H_4-$ | 0 | 1 | H | $COCH_3$ | base | 100/0 | 122-124 |
| 35 | $3-Cl-C_6H_4-$ | 0 | 1 | H | $COOCH_3$ | base | 100/0 | 72-74 |
| 36 | $3-Cl-C_6H_4-$ | 0 | 2 | H | $COCH_3$ | base | 100/0 | 112-114 |
| 37 | $3-Cl-C_6H_4-$ | 0 | 2 | H | $COOCH_3$ | base | 100/0 | 88-90 |
| 38 | $3-Cl-C_6H_4-$ | 0 | 3 | H | $COCH_3$ | base | 100/0 | 93-95 |
| 39 | $3-Cl-C_6H_4-$ | 0 | 3 | H | $COOCH_3$ | base | 100/0 | 46-48 |
| 40 | $3-Cl-C_6H_4-$ | 1 | 1 | H | $COCH_3$ | base | 90/10 | 121-122 |
| 41 | $3-Cl-C_6H_4-$ | 1 | 2 | H | $COCH_3$ | base | 80/20 | 110-111 |
| 42 | $3-Cl-C_6H_4-$ | 1 | 3 | H | $COCH_3$ | base | 85/15 | 80-82 |

Tableau (suite)

| N° | Ar | m | n | $R_1$ | $R_2$ | Sel | T/C | F (°C) |
|---|---|---|---|---|---|---|---|---|
| 43 | 4-Cl-$C_6H_4$- | 0 | 1 | H | H | HCl | 100/0 | 270-272 |
| 44 | 4-Cl-$C_6H_4$- | 0 | 1 | H | CHO | base | 100/0 | 112-114 |
| 45 | 4-Cl-$C_6H_4$- | 0 | 1 | H | $COCH_3$ | base | 100/0 | 112-114 |
| 46 | 4-Cl-$C_6H_4$- | 0 | 1 | H | $COOCH_3$ | base | 100/0 | 102-103 |
| 47 | 4-Cl-$C_6H_4$- | 0 | 1 | $CH_3$ | $CH_3$ | HCl | 100/0 | 238-240 |
| 48 | 4-Cl-$C_6H_4$- | 0 | 1 | $CH_3$ | $COOCH_3$ | base | 100/0 | 60-62 |
| 49 | 4-Cl-$C_6H_4$- | 0 | 2 | H | $COCH_3$ | base | 100/0 | 162-163 |
| 50 | 4-Cl-$C_6H_4$- | 0 | 2 | H | $COOCH_3$ | base | 100/0 | 118-120 |
| 51 | 4-Cl-$C_6H_4$- | 0 | 3 | H | $CH_3$ | HCl | 100/0 | 136-138 |
| 52 | 4-Cl-$C_6H_4$- | 0 | 3 | H | CHO | base | 100/0 | 114-116 |
| 53 | 4-Cl-$C_6H_4$- | 0 | 3 | H | $COCH_3$ | base | 100/0 | 103-105 |
| 54 | 4-Cl-$C_6H_4$- | 0 | 3 | H | $COOCH_3$ | base | 100/0 | 88-90 |
| 55 | 4-Cl-$C_6H_4$- | 0 | 3 | $CH_3$ | $COCH_3$ | base | 100/0 | 56-58 |
| 56 | 4-Cl-$C_6H_4$- | 0 | 4 | H | $COCH_3$ | base | 100/0 | 144-145 |
| 57 | 4-Cl-$C_6H_4$- | 0 | 4 | $CH_3$ | $CH_3$ | HCl | 100/0 | 195-196 |
| 58 | 4-Cl-$C_6H_4$- | 1 | 1 | H | $COCH_3$ | base | 60/40 | 95 |

Tableau (suite)

| N° | Ar | m | n | $R_1$ | $R_2$ | Sel | T/C | F (°C) |
|---|---|---|---|---|---|---|---|---|
| 59 | 4-Cl-$C_6H_4$- | 1 | 2 | H | $COOCH_3$ | base | 85/15 | 84-86 |
| 60 | 4-Cl-$C_6H_4$- | 1 | 3 | H | $COCH_3$ | base | 60/40 | 100 |
| 61 | 3,4-$Cl_2$-$C_6H_3$- | 0 | 1 | H | $COCH_3$ | base | 100/0 | 136-137 |
| 62 | 3,4-$Cl_2$-$C_6H_3$- | 0 | 3 | H | $COCH_3$ | base | 100/0 | 131-132 |
| 63 | 4-Br-$C_6H_4$- | 0 | 2 | H | $COCH_3$ | base | 100/0 | 162-163 |
| 64 | 4-Br-$C_6H_4$- | 0 | 3 | H | $COCH_3$ | base | 100/0 | 112-114 |
| 65 | 2-$CH_3$-$C_6H_4$- | 0 | 1 | H | $COCH_3$ | base | 100/0 | 129-130 |
| 66 | 2-$CH_3$-$C_6H_4$- | 0 | 3 | H | $COCH_3$ | base | 100/0 | 118-120 |
| 67 | 2-$CH_3$-$C_6H_4$- | 0 | 3 | $CH_3$ | $COCH_3$ | base | 100/0 | $n_D^{23}$ =1,529 |
| 68 | 2-$CH_3$-$C_6H_4$- | 1 | 3 | H | $COCH_3$ | base | 80/20 | 52-54 |
| 69 | 3-$CH_3$-$C_6H_4$- | 0 | 1 | H | $COCH_3$ | base | 100/0 | 120-121 |
| 70 | 3-$CH_3$-$C_6H_4$- | 0 | 3 | H | $COCH_3$ | base | 100/0 | 72-73 |
| 71 | 3-$CH_3$-$C_6H_4$- | 1 | 1 | H | $COCH_3$ | base | 90/10 | 92-93 |
| 72 | 3-$CH_3$-$C_6H_4$- | 1 | 2 | H | $COCH_3$ | base | 100/0 | 99-100 |
| 73 | 3-$CH_3$-$C_6H_4$- | 1 | 3 | H | $COCH_3$ | base | 100/0 | 86-87 |
| 74 | 4-$CH_3$-$C_6H_4$- | 0 | 1 | H | $COCH_3$ | base | 100/0 | 134-135 |

Tableau (suite)

| N° | Ar | m | n | $R_1$ | $R_2$ | Sel | T/C | F (°C) |
|---|---|---|---|---|---|---|---|---|
| 75 | $4-CH_3-C_6H_4-$ | 0 | 1 | $CH_3$ | $CH_3$ | HCl | 100/0 | 234-235 |
| 76 | $4-CH_3-C_6H_4-$ | 0 | 3 | H | $COCH_3$ | base | 100/0 | 124-125 |
| 77 | $4-CH_3-C_6H_4-$ | 1 | 1 | H | $COCH_3$ | base | 50/50 | 89-90 |
| 78 | $4-CH_3-C_6H_4-$ | 1 | 2 | H | $COCH_3$ | base | 100/0 | 137-138 |
| 79 | $4-CH_3-C_6H_4-$ | 1 | 3 | H | $COCH_3$ | base | 100/0 | 122-123 |
| 80 | $4-tC_4H_9-C_6H_4-$ | 1 | 1 | H | $COCH_3$ | base | 50/50 | $n_D^{26}$ =1,5040 |
| 81 | $4-tC_4H_9-C_6H_4-$ | 1 | 3 | H | $COCH_3$ | base | 60/40 | 46-48 |
| 82 | $2,3-(CH_3)_2-C_6H_3-$ | 1 | 1 | H | $COCH_3$ | base | 80/20 | 114-116 |
| 83 | $2,3-(CH_3)_2-C_6H_3-$ | 1 | 2 | H | $COCH_3$ | base | 100/0 | 120-121 |
| 84 | $2,3-(CH_3)_2-C_6H_3-$ | 1 | 3 | H | $COCH_3$ | base | 90/10 | 89-90 |
| 85 | $2,5-(CH_3)_2-C_6H_3-$ | 1 | 1 | H | $COCH_3$ | base | 100/0 | 107-108 |
| 86 | $2,5-(CH_3)_2-C_6H_3-$ | 1 | 1 | H | $COOCH_3$ | base | 100/0 | 84-85 |
| 87 | $2,5-(CH_3)_2-C_6H_3-$ | 1 | 2 | H | $COCH_3$ | base | 85/15 | 97-98 |
| 88 | $2,5-(CH_3)_2-C_6H_3-$ | 1 | 3 | H | $COCH_3$ | base | 100/0 | 95-96 |
| 89 | $2-CH_3O-C_6H_4-$ | 0 | 1 | H | $COCH_3$ | base | 90/10 | 96-98 |
| 90 | $2-CH_3O-C_6H_4-$ | 0 | 2 | H | $COCH_3$ | base | 90/10 | 88-90 |

Tableau (suite)

| N° | Ar | m | n | $R_1$ | $R_2$ | Sel | T/C | F (°C) |
|---|---|---|---|---|---|---|---|---|
| 91 | $2-CH_3O-C_6H_4-$ | 0 | 3 | H | $COCH_3$ | base | 100/0 | 98-100 |
| 92 | $2-CH_3O-C_6H_4-$ | 1 | 1 | H | $COCH_3$ | base | 60/40 | 82-83 |
| 93 | $2-CH_3O-C_6H_4-$ | 1 | 2 | H | $COCH_3$ | base | 90/10 | 87-89 |
| 94 | $2-CH_3O-C_6H_4-$ | 1 | 3 | H | $COCH_3$ | base | 95/5 | 96-98 |
| 95 | $3-CH_3O-C_6H_4-$ | 0 | 1 | H | $COCH_3$ | base | 90/10 | 90-92 |
| 96 | $3-CH_3O-C_6H_4-$ | 0 | 2 | H | $COCH_3$ | base | 90/10 | 88-90 |
| 97 | $3-CH_3O-C_6H_4-$ | 0 | 3 | H | $COCH_3$ | base | 85/15 | 88-90 |
| 98 | $3-CH_3O-C_6H_4-$ | 1 | 2 | H | $COCH_3$ | base | 90/10 | 108-110 |
| 99 | $3-CH_3O-C_6H_4-$ | 1 | 3 | H | $COCH_3$ | base | 75/25 | 100-102 |
| 100 | $4-CH_3O-C_6H_4-$ | 0 | 1 | H | H | HCl | 100/0 | 247-248 |
| 101 | $4-CH_3O-C_6H_4-$ | 0 | 1 | H | $COCH_3$ | base | 100/0 | 117,5-118 |
| 102 | $4-CH_3O-C_6H_4-$ | 0 | 1 | $CH_3$ | $CH_3$ | HCl | 100/0 | 210 |
| 103 | $4-CH_3O-C_6H_4-$ | 0 | 2 | $CH_3$ | $CH_3$ | HCl | 100/0 | 168-170 |
| 104 | $4-CH_3O-C_6H_4-$ | 0 | 3 | H | $COCH_3$ | base | 100/0 | 99-99,5 |
| 105 | $4-CH_3O-C_6H_4-$ | 0 | 3 | H | $COC_2H_5$ | base | 100/0 | 79-80 |
| 106 | $4-CH_3O-C_6H_4-$ | 0 | 4 | $CH_3$ | $CH_3$ | HCl | 100/0 | 175-178 |

Tableau (suite)

| N° | Ar | m | n | $R_1$ | $R_2$ | Sel | T/C | F (°C) |
|---|---|---|---|---|---|---|---|---|
| 107 | $4-CH_3O-C_6H_4-$ | 1 | 1 | H | $COCH_3$ | base | 90/10 | 85-86 |
| 108 | $4-CH_3O-C_6H_4-$ | 1 | 2 | H | $COCH_3$ | base | 65/35 | 89-90 |
| 109 | $4-CH_3O-C_6H_4-$ | 1 | 3 | H | $COCH_3$ | base | 50/50 | 65-66 |
| 110 | $3,4-(CH_3O)_2-C_6H_3-$ | 0 | 1 | H | $COCH_3$ | base | 100/0 | 134-136 |
| 111 | $3,4-(CH_3O)_2-C_6H_3-$ | 0 | 3 | H | $COCH_3$ | base | 100/0 | 115-116 |
| 112 | $3-CF_3-C_6H_4-$ | 0 | 3 | H | $COCH_3$ | base | 100/0 | 72-74 |
| 113 | $3-CF_3-C_6H_4-$ | 1 | 1 | H | $COCH_3$ | base | 70/30 | 117-118 |
| 114 | $3-CF_3-C_6H_4-$ | 1 | 1 | H | $COOCH_3$ | base | 65/35 | 58-60 |
| 115 | $3-CF_3-C_6H_4-$ | 1 | 3 | H | $COCH_3$ | base | 100/0 | 89-90 |
| 116 | $3-CF_3-4-F-C_6H_3-$ | 1 | 2 | H | $COCH_3$ | base | 60/40 | 58-60 |
| 117 | $3-CF_3-4-F-C_6H_3-$ | 1 | 3 | H | $COCH_3$ | base | 55/45 | 39-43 |
| 118 | $C_{10}H_7-(1)-$ | 0 | 1 | H | $COCH_3$ | base | 100/0 | 140-142 |
| 119 | $C_{10}H_7-(1)-$ | 0 | 2 | H | $COCH_3$ | base | 80/20 | 136-139 |
| 120 | $C_{10}H_7-(1)-$ | 0 | 3 | H | H | HCl | 100/0 | 191-192 |
| 121 | $C_{10}H_7-(1)-$ | 0 | 3 | H | $CH_3$ | HCl | 100/0 | 142-144 |
| 122 | $C_{10}H_7-(1)-$ | 0 | 3 | H | CHO | base | 100/0 | 108-110 |

Tableau (suite)

| N° | Ar | m | n | $R_1$ | $R_2$ | Sel | T/C | F (°C) |
|---|---|---|---|---|---|---|---|---|
| 123 | $C_{10}H_7$-(1)- | 0 | 3 | H | $COCH_3$ | base | 100/0 | 158-160 |
| 124 | $C_{10}H_7$-(1)- | 0 | 3 | H | $COOCH_3$ | base | 100/0 | 80-82 |
| 125 | $C_{10}H_7$-(1)- | 0 | 3 | H | $COOC_2H_5$ | base | 100/0 | 93-94 |
| 126 | $C_{10}H_7$-(1)- | 0 | 3 | H | $COOiC_3H_7$ | base | 100/0 | 118-120 |
| 127 | $C_{10}H_7$-(1)- | 0 | 3 | H | $COOiC_4H_9$ | base | 100/0 | 78-80 |
| 128 | $C_{10}H_7$-(1)- | 0 | 3 | $CH_3$ | $COCH_3$ | base | 100/0 | $n_D^{21}$ =1,5752 |
| 129 | $C_{10}H_7$-(1)- | 1 | 1 | H | $COCH_3$ | base | 60/40 | 136 |
| 130 | $C_{10}H_7$-(1)- | 1 | 2 | H | $COOCH_3$ | base | 100/0 | 86 |
| 131 | $C_{10}H_7$-(1)- | 1 | 3 | H | $COCH_3$ | base | 60/40 | 105 |
| 132 | 4-Cl-$C_{10}H_6$-(1)- | 0 | 1 | H | $COCH_3$ | base | 100/0 | 188-190 |
| 133 | 4-Cl-$C_{10}H_6$-(1)- | 0 | 3 | H | $COCH_3$ | base | 100/0 | 140-142 |
| 134 | 6-Cl-$C_{10}H_6$-(1)- | 0 | 1 | H | $COCH_3$ | base | 100/0 | 166-168 |
| 135 | 6-Cl-$C_{10}H_6$-(1)- | 0 | 3 | H | $COCH_3$ | base | 100/0 | 160-162 |
| 136 | 6-$CH_3O$-$C_{10}H_6$-(1)- | 0 | 1 | H | $COCH_3$ | base | 100/0 | 158 |
| 137 | 6-$CH_3O$-$C_{10}H_6$-(1)- | 0 | 1 | H | $COOCH_3$ | base | 100/0 | 142-144 |
| 138 | 6-$CH_3O$-$C_{10}H_6$-(1)- | 0 | 3 | H | $COCH_3$ | base | 100/0 | 146 |

Tableau (suite et fin)

| N° | Ar | m | n | $R_1$ | $R_2$ | Sel | T/C | F (°C) |
|---|---|---|---|---|---|---|---|---|
| 139 | $7-CH_3O-C_{10}H_6-(1)-$ | 0 | 1 | H | $COCH_3$ | base | 90/10 | 138-140 |
| 140 | $7-CH_3O-C_{10}H_6-(1)-$ | 0 | 3 | H | $COCH_3$ | base | 100/0 | 132-134 |
| 141 | $6-cC_3H_5CH_2O-C_{10}H_6-(1)-$ | 0 | 3 | H | $COCH_3$ | base | 90/10 | 124-126 |
| 142 | $C_{10}H_7-(2)-$ | 0 | 1 | H | $COCH_3$ | base | 100/0 | 138-140 |
| 143 | $C_{10}H_7-(2)-$ | 0 | 3 | H | $COCH_3$ | base | 100/0 | 135-136 |
| 144 | $C_{10}H_7-(2)-$ | 1 | 1 | H | $COCH_3$ | base | 60/40 | 116 |
| 145 | $C_{10}H_7-(2)-$ | 1 | 3 | H | $COCH_3$ | base | 100/0 | 106 |
| 146 | $6-CH_3-C_{10}H_6-(2)-$ | 0 | 1 | H | $COCH_3$ | base | 100/0 | 180-182 |
| 147 | $6-CH_3-C_{10}H_6-(2)-$ | 0 | 3 | H | $COCH_3$ | base | 85/15 | 140-142 |
| 148 | $6-CH_3O-C_{10}H_6-(2)-$ | 0 | 1 | H | $COCH_3$ | base | 90/10 | 132-134 |
| 149 | $6-CH_3O-C_{10}H_6-(2)-$ | 0 | 3 | H | $COCH_3$ | base | 95/5 | 126-128 |
| 150 | $6-cC_3H_5CH_2O-C_{10}H_6-(2)-$ | 0 | 3 | H | $COCH_3$ | base | 100/0 | 188-120 |

Les composés de l'invention ont fait l'objet d'essais pharmacologiques qui ont mis en évidence leurs propriétés thérapeutiques.

Ainsi leur activité anticonvulsivante potentielle a été testée dans différents modèles expérimentaux chez la souris (convulsions provoquées par électrochoc maximal et mortalité induite soit par la bicuculline, soit par la strychnine), selon les protocoles décrits par E. A. Swinyard et J. H. Woodhead dans "Experimental detection, quantification and evaluation of anticonvulsants", *in* Antiepileptic Drugs, D. M. Woodbury, J. K. Penry and C. E. Pippenger (Eds), Raven Press, New York, pp. 111-125 (1982).

Dans ces modèles, les composés les plus actifs ont une $DA_{50}$ (dose active qui inhibe de 50% les convulsions ou la mortalité induites par les différents agents convulsivants) de l'ordre de 20 mg/kg par la voie intrapéritonéale, et de l'ordre de 40 mg/kg par la voie orale.

Les composés ont également fait l'objet d'un autre essai, décrit par C. Fleury dans "Nouvelle technique pour mesurer l'effort musculaire de la souris, dite test de l'agrippement", *in* Arch. Sci. (Geneva), **10**, 107-113 (1957).
Les composés les plus actifs sont, dans cet essai, sans effets à la dose maximale testée de 600 mg/kg par voie orale.

Enfin un autre essai, dit test du rota rod, décrit par N. W. Dunham et T. S. Miya dans "A note on a simple apparatus for detecting neurological deficit in rats and mice", *in* J. Am. Pharm. Assoc., **46**, 208-209 (1957), a montré que les composés de l'invention ne présentent pas de signes neurotoxiques à la dose maximale testée de 400 mg/kg par voie orale.

Les résultats des essais montrent que les composés de l'invention, par leurs propriétés anticonvulsivantes, peuvent être utilisés pour le traitement de l'épilepsie. Le traitement d'autres affections, telles que la spasticité, les dyskinésies, la dépression, peut également être envisagé.

A cet effet ils peuvent être présentés sous toutes formes galéniques permettant leur administration entérale ou parentérale, en association avec des excipients appropriés, telles que comprimés, dragées, capsules, gélules, suppositoires, solutions et suspensions buvables ou injectables, dosées pour permettre une posologie de 5 à 15 mg/kg de substance active par jour.

**Revendications**

**Revendications pour les Etats contractants suivants : AT, BE, CH, DE, DK, FR, GB, IT, LI, LU, NL, SE**

1. Composé répondant à la formule générale (I)

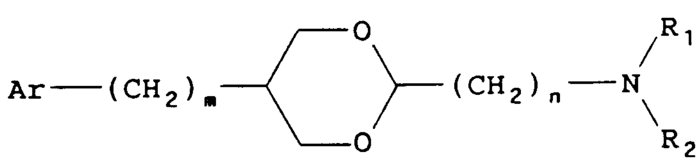

(I)

dans laquelle
m représente 0 ou 1,
n représente 1, 2, 3 ou 4,
$R_1$ représente soit un atome d'hydrogène soit un groupe méthyle,
$R_2$ représente soit un atome d'hydrogène, soit un groupe méthyle, soit un groupe alcanoyle de formule générale COR' dans laquelle R' représente un atome d'hydrogène ou un groupe alkyle en $C_1$-$C_4$ linéaire ou ramifié, soit un groupe alcoxy carbonyle de formule générale COOR" dans laquelle R" représente un groupe alkyle en $C_1$-$C_4$ linéaire ou ramifié, et Ar représente soit un groupe phényle portant éventuellement un ou deux substituants choisis parmi les atomes d'halogène et les groupes alkyle en $C_1$-$C_4$, méthoxy et trifluorométhyle, soit un groupe naphtalén-1-yle ou naphtalén-2-yle portant éventuellement un substituant choisi parmi les atomes d'halogène et les groupes méthyle, méthoxy et cyclopropylméthoxy, sous forme de stéréoisomères cis ou trans, et sous forme de bases libres ou de sels d'addition à des acides.

2. Composé selon la revendication 1, caractérisés en ce que Ar représente un groupe naphtalén-1-yle ou naphtalén-2-yle éventuellement substitué.

3. Composé selon la revendication 1, en l'espèce le *N*-[3-[5-(naphtalén-1-yl)-1,3-dioxan-2-yl]propyl]-acétamide.

4. Composé selon la revendication 1, en l'espèce le *N*-[3-[5-(6-méthoxynaphtalén-1-yl)-1,3-dioxan-2-yl]-propyl]acétamide.

5. Composé selon la revendication 1, en l'espace le *N*-[3-[5-[6-(cyclopropylméthoxy)naphtalén-1-yl]-1,3-dioxan-2-yl]propyl]acétamide.

6. Procédé de préparation d'un composé selon l'une des revendications 1 à 5, caractérisé en ce qu'on fait réagir un diol de formule générale (II)

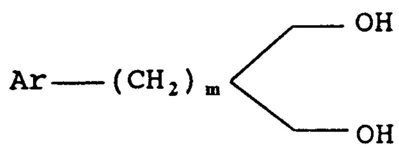

(II)

(dans laquelle m et Ar sont tels que définis dans la revendication 1) avec un acétal de formule générale (III)

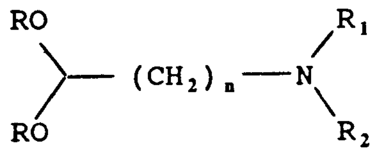

(III)

(dans laquelle n, $R_1$ et $R_2$ sont tels que définis dans la revendication 1 et R représente un groupe alkyle en $C_1$-$C_4$).

**7.** Médicament, caractérisé en ce qu'il consiste en un composé selon l'une des revendications 1 à 5.

**8.** Composition pharmaceutique, caractérisée en ce qu'elle contient un composé selon l'une des revendications 1 à 5, associé à un excipient pharmaceutique.

**Revendications pour les Etats contractants suivants : ES, GR**

**1.** Procédé de préparation d'un composé répondant à la formule générale (I)

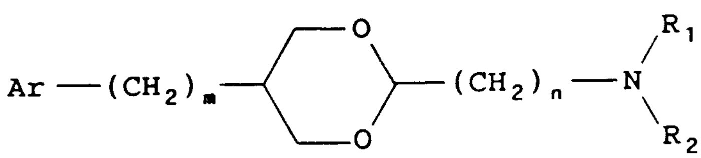

(I)

dans laquelle
m représente 0 ou 1,
n représente 1, 2, 3 ou 4,
$R_1$ représente soit un atome d'hydrogène soit un groupe méthyle,
$R_2$ représente soit un atome d'hydrogène, soit un groupe méthyle, soit un groupe alcanoyle de formule générale COR' dans laquelle R' représente un atome d'hydrogène ou un groupe alkyle en $C_1$-$C_4$ linéaire ou ramifié, soit un groupe alcoxy carbonyle de formule générale COOR'' dans laquelle R'' représente un groupe alkyle en $C_1$-$C_4$ linéaire ou ramifié, et Ar représente soit un groupe phényle portant éventuellement un ou deux substituants choisis parmi les atomes d'halogène et les groupes alkyle en $C_1$-$C_4$, méthoxy et trifluorométhyle, soit un groupe naphtalén-1-yle ou naphtalén-2-yle portant éventuellement un substituant choisi parmi les atomes d'halogène et les groupes méthyle, méthoxy et cyclopropylméthoxy, procédé caractérisé en ce qu'on fait réagir un diol de formule générale (II)

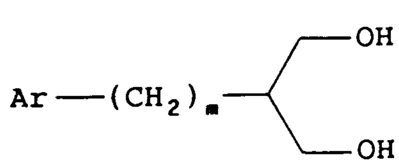

(II)

(dans laquelle m et Ar sont tels que définis ci-dessus) avec un acétal de formule générale (III)

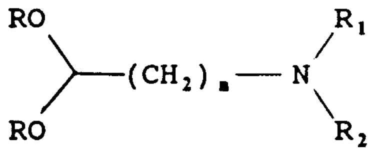

(III)

(dans laquelle n, $R_1$ et $R_2$ sont tels que définis ci-dessus et R représente un groupe alkyle en $C_1$-$C_4$).

**2.** Procédé selon la revendication 1, caractérisés en ce que Ar représente un groupe naphtalén-1-yle ou naphtalén-2-yle éventuellement substitué.

**3.** Procédé selon la revendication 1, caractérisé en ce qu'on prépare le *N*-[3-[5-(naphtalén-1-yl)-1,3-dioxan-2-yl]propyl]acétamide.

**4.** Procédé selon la revendication 1, caractérisé en ce qu'on prépare le *N*-[3-[5-(6-méthoxynaphtalén-1-yl)-1,3-dioxan-2-yl]propyl]acétamide.

**5.** Procédé selon la revendication 1, caractérisé en ce qu'on prépare le *N*-[3-[5-[6-(cyclopropylméthoxy)-naphtalén-1-yl]-1, 3-dioxan-2-yl]propyl]acétamide.

**Claims**

**Claims for the following Contracting States : AT, BE, CH, DE, DK, FR, GB, IT, LI, LU, NL, SE**

**1.** Compound corresponding to the general formula (I)

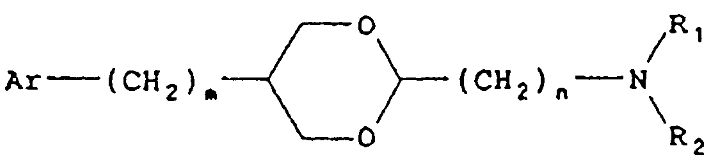

(I)

in which
m represents 0 or 1,
n represents 1, 2, 3 or 4,
$R_1$ represents either a hydrogen atom or a methyl group,
$R_2$ represents either a hydrogen atom, or a methyl group, or an alkanoyl group of the general formula COR' in which R' represents a hydrogen atom or a linear or branched $C_1$-$C_4$-alkyl group, or an alkoxycarbonyl group of the general formula COOR" in which R" represents a linear or branched $C_1$-$C_4$-alkyl group, and
Ar represents either a phenyl group optionally carrying one or two substituents chosen from amongst halogen atoms and $C_1$-$C_4$-alkyl, methoxy and trifluoromethyl groups, or a naphthalen-1-yl or naphthalen-2-yl group optionally carrying a substituent chosen from amongst halogen atoms and methyl, methoxy and cyclopropylmethoxy groups,
in the form of cis- or trans-stereoisomers, and in the form of free bases or of acid addition salts.

**2.** Compound according to Claim 1, characterized in that Ar represents an optionally substituted naphthalen-1-yl or naphthalen-2-yl group.

**3.** Compound according to Claim 1, named *N*-[3-[5-(naphthalen-1-yl)-1,3-dioxan-2-yl]propyl]acetamide.

**4.** Compound according to Claim 1, named *N*-[3-[5-(6-methoxynaphthalen-1-yl)-1,3-dioxan-2-yl]propyl]-acetamide.

**5.** Compound according to Claim 1, named *N*-[3-[5-[6-(cyclopropylmethoxy)naphthalen-1-yl]-1,3-dioxan-2-yl]propyl]acetamide.

6. Process for the preparation of a compound according to one of Claims 1 to 5, characterized in that a diol of the general formula (II)

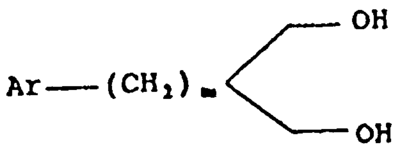

(II)

(in which m and Ar are as defined in Claim 1) is reacted with an acetal of the general formula (III)

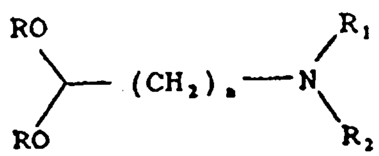

(III)

(in which n, $R_1$ and $R_2$ are as defined in Claim 1 and R represents a $C_1$-$C_4$-alkyl group).

7. Medicament, characterized in that it consists of a compound according to one of Claims 1 to 5.

8. Pharmaceutical composition, characterized in that it contains a compound according to one of Claims 1 to 5, associated with a pharmaceutical excipient.

**Claims for the following Contracting States : ES, GR**

1. Process for the preparation of a compound corresponding to the general formula (I)

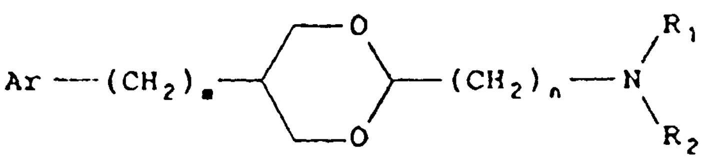

(I)

in which
m represents 0 or 1,
n represents 1, 2, 3 or 4,
$R_1$ represents either a hydrogen atom or a methyl group,
$R_2$ represents either a hydrogen atom, or a methyl group, or an alkanoyl group of the general formula COR' in which R' represents a hydrogen atom or a linear or branched $C_1$-$C_4$-alkyl group, or an alkoxycarbonyl group of the general formula COOR'' in which R'' represents a linear or branched $C_1$-$C_4$-alkyl group, and
Ar represents either a phenyl group optionally carrying one or two substituents chosen from amongst halogen atoms and $C_1$-$C_4$-alkyl, methoxy and trifluoromethyl groups, or a naphthalen-1-yl or naphthalen-2-yl group optionally carrying a substituent chosen from amongst halogen atoms and methyl, methoxy and cyclopropylmethoxy groups, characterized in that a diol of the general formula (II)

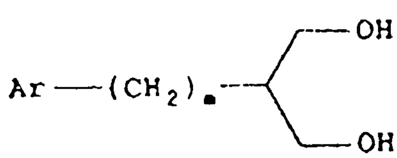

(II)

(in which m and Ar are as defined above) is reacted with an acetal of the general formula (III)

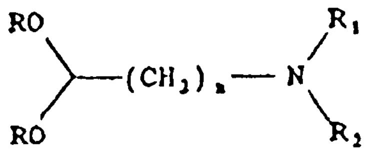

(III)

(in which n, $R_1$ and $R_2$ are as defined above and R represents a $C_1$-$C_4$-alkyl group).

2. Process according to Claim 1, characterized in that Ar represents an optionally substituted naphthalen-1-yl or naphthalen-2-yl group.

3. Process according to Claim 1, characterized in that *N*-[3-[5-(naphthalen-1-yl)-1,3-dioxan-2-yl]propyl]-acetamide is prepared.

4. Process according to Claim 1, characterized in that *N*-[3-[5-(6-methoxynaphthalen-1-yl)-1,3-dioxan-2-yl]-propyl]acetamide is prepared.

5. Process according to Claim 1, characterized in that *N*-[3-5-[6-cyclopropylmethoxy)naphthalen-1-yl]-1,3-dioxan-2-yl]propyl]acetamide is prepared.

**Patentansprüche**

**Patentansprüche für folgende Vertragsstaaten : AT, BE, CH, DE, DK, FR, GB, IT, LI, LU, NL, SE**

1. Verbindung der allgemeinen Formel (I)

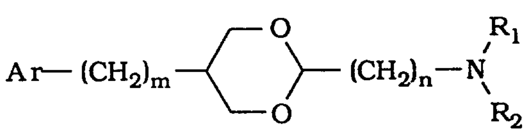

(I)

in der
m 0 oder 1,
n 1, 2, 3 oder 4,
$R_1$ entweder ein Wasserstoffatom oder eine Methylgruppe,
$R_2$ entweder ein Wasserstoffatom oder eine Methylgruppe oder eine Alkanoylgruppe der allgemeinen Formel COR', worin R' ein Wasserstoffatom oder eine geradkettige oder verzweigte $C_1$-$C_4$-Alkylgruppe darstellt, oder eine Alkoxycarbonylgruppe der allgemeinen Formel COOR", worin R" eine geradkettige oder verzweigte $C_1$-$C_4$-Alkylgruppe darstellt, und
Ar entweder eine Phenylgruppe, die gegebenenfalls einen oder zwei Substituenten ausgewählt aus Halogenatomen, $C_1$-$C_4$-Alkylgruppen, Methoxygruppen und Trifluormethylgruppen substituiert ist, oder eine Naphthalin-1-yl- oder Naphthalin-2-yl-gruppe, die gegebenenfalls einen Substituenten ausgewählt aus Halogenatomen, Methylgruppen, Methoxygruppen und Cyclopropylmethoxygruppen trägt, bedeuten,
in Form der cis- oder trans-Stereoisomeren und in Form der freien Basen oder der Additionssalze mit Säuren.

2. Verbindung nach Anspruch 1, **dadurch gekennzeichnet**, daß Ar eine gegebenenfalls substituierte Naphthalin-1-yl- oder Naphthalin-2-yl-gruppe bedeutet.

3. Verbindung nach Anspruch 1, nämlich N-[3-[5-(Naphthalin-1-yl)-1,3-dioxan-2-yl]-propyl]-acetamid.

4. Verbindung nach Anspruch 1, nämlich N-[3-[5-(6-Methoxynaphthalin-1-yl)-1,3-dioxan-2-yl]-propyl]-acetamid.

5. Verbindung nach Anspruch 1, nämlich N-[3-[5-[6-(Cyclopropylmethoxy)naphthalin-1-yl]-1,3-dioxan-2-yl]-propyl]-acetamid.

6. Verfahren zur Herstellung einer Verbindung nach einem der Ansprüche 1 bis 5, **dadurch gekennzeichnet**, daß man ein Diol der allgemeinen Formel (II)

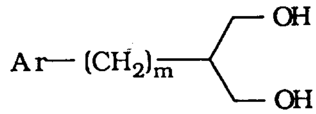

(II)

(in der m und Ar die in Anspruch 1 angegebenen Bedeutungen besitzen) mit einem Acetal der allgemeinen Formel (III)

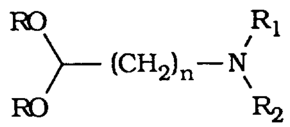

(III)

(in der n, $R_1$ und $R_2$ die in Anspruch 1 angegebenen Bedeutungen besitzen und R eine $C_1$-$C_4$-Alkylgruppe darstellt) umsetzt.

7. Arzneimittel, **dadurch gekennzeichnet**, daß es aus einer Verbindung nach einem der Ansprüche 1 bis 5 besteht.

8. Pharmazeutische Zubereitung, **dadurch gekennzeichnet**, daß sie eine Verbindung nach einem der Ansprüche 1 bis 5 in Kombination mit einem pharmazeutischen Trägermaterial enthält.

**Patentansprüche für folgende Vertragsstaaten : ES, GR**

1. Verfahren zur Herstellung einer Verbindung der allgemeinen Formel (I)

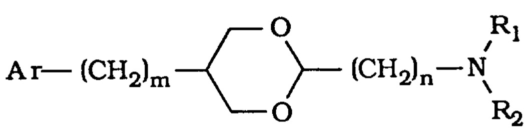

(I)

in der
m 0 oder 1,
n 1, 2, 3 oder 4,
$R_1$ entweder ein Wasserstoffatom oder eine Methylgruppe,
$R_2$ entweder ein Wasserstoffatom oder eine Methylgruppe oder eine Alkanoylgruppe der allgemeinen Formel COR', worin R' ein Wasserstoffatom oder eine geradkettige oder verzweigte $C_1$-$C_4$-Alkylgruppe darstellt, oder eine Alkoxycarbonylgruppe der allgemeinen Formel COOR'', worin R'' eine geradkettige oder verzweigte $C_1$-$C_4$-Alkylgruppe darstellt, und
Ar entweder eine Phenylgruppe, die gegebenenfalls einen oder zwei Substituenten ausgewählt aus Halogenatomen, $C_1$-$C_4$-Alkylgruppen, Methoxygruppen und Trifluormethylgruppen substituiert ist, oder eine Naphthalin-1-yl- oder Naphthalin-2-yl-gruppe, die gegebenenfalls einen Substituenten ausgewählt aus Halogenatomen, Methylgruppen, Methoxygruppen und Cyclopropylmethoxygruppen trägt, bedeuten,
**dadurch gekennzeichnet**, daß man ein Diol der allgemeinen Formel (II)

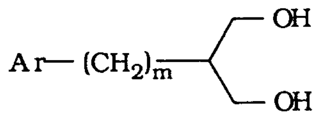

(II)

(in der m und Ar die oben angegebenen Bedeutungen besitzen) mit einem Acetal der allgemeinen Formel (III)

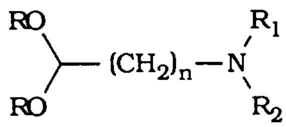

(III)

(in der n, $R_1$ und $R_2$ die oben angegebenen Bedeutungen besitzen und R eine $C_1$-$C_4$-Alkylgruppe darstellt) umsetzt.

2. Verfahren nach Anspruch 1, **dadurch gekennzeichnet**, daß Ar eine gegebenenfalls substituierte Naphthalin-1-yl- oder Naphthalin-2-yl-gruppe bedeutet.

3. Verfahren nach Anspruch 1, **dadurch gekennzeichnet**, daß man N-[3-[5-(Naphthalin-1-yl)-1,3-dioxan-2-yl]-propyl]-acetamid herstellt.

4. Verfahren nach Anspruch 1, **dadurch gekennzeichnet**, daß man N-[3-[5-(6-Methoxynaphthalin-1-yl)-1,3-dioxan-2-yl]-propyl]-acetamid herstellt.

5. Verfahren nach Anspruch 1, **dadurch gekennzeichnet**, daß man N-[3-[5-[6-(Cyclopropylmethoxy)-naphthalin-1-yl]-1,3-dioxan-2-yl]-propyl]-acetamid herstellt.